# EUROPEAN PATENT APPLICATION

(11) **EP 1 110 547 A2**
(43) Date of publication of application: **27.06.2001**
(21) Application number: 00128164.1
(22) Date of filing: 21.12.2000
(51) Int. Cl.: A61K 9/72

(54) **Helium and neon as means delivering drug in inhaler**

(30) Priority: 23.12.1999 US 471089
(71) Applicant: PRAXAIR TECHNOLOGY, INC., Danbury, CT 06810-5113 (US)
(72) Inventor: Cheng, Alan, Livingston, New Jersey 07039 (US); Fishman, Royce S., Hillsdale, New Jersey 07642 (US)
(74) Representative: Schwan - Schwan - Schorer

(57) **Abstract**

An aerosol inhaler is disclosed with a gas propellant containing more than 80% helium or neon. Also disclosed is a method of treating a subject with a pharmaceutical agent, comprising administering the pharmaceutical agent to the subject's lungs using an aerosol inhaler containing a gas propellant, wherein said a gas propellant contains more than 80% helium or neon in order to improve the laminae flow from the inhaler to the lungs.

## Description

### FIELD OF THE INVENTION

This invention relates to the use of helium and neon as predominant carrier medium or propellant for delivering a pharmaceutically active agent to a targeted tissue, wherein the pharmaceutically active agent is suspended in the carrier medium or propellant. More specifically, the pharmaceutically active agent is delivered to a patient's lungs via an aerosol inhaler. Even more specifically the carrier medium or propellant may consist of substantially pure helium.

### BACKGROUND OF THE INVENTION

Delivery of therapeutic drugs via the lungs for respiratory and non-respiratory systemic diseases, is increasingly being recognized as a viable if not superior alternative to administration of drugs orally/nasally, rectally, transdermally, by intravenous needle injection, intra-muscular needle injection, or gas jet driven needle-less injection through the skin and into the muscle.

Pulmonary drug delivery can, dependent on the drug and disease: improve the efficacy of a drug; improve the bioavailability of a drug which is particularly important for biological compounds such as peptides and proteins; improve targeting to an organ or receptor site, thus reducing unwanted side effects (which is an important consideration, for example, with anticancer agents); and mimic the biopattern of a disease, or circadian rhythm, e.g., as in the case of sustained-release anti-hypertensives designed to peak coinciding with the early morning blood pressure surge.

Recognition of the ability to deliver systemic therapeutic drugs by inhalation due to the above physiological properties of the lung and circulatory system, has led to a large number of different therapeutic drugs being developed and evaluated for administration by inhalation to treat non-respiratory diseases.

There are different optimal delivery sites within the bronchial tree, depending on whether one is treating respiratory or systemic diseases. For example, drugs such as bronchodilators, to treat a respiratory disease such as asthma need to be deposited in large and small airways. However, therapeutic drugs to treat other systemic diseases, like insulin, need to be deposited in the terminal bronchioles and the alveoli.

A key need is maximizing the number of these smallest particles that are delivered to the terminal branches of the bronchioles and the alveoli. Small particles, typically 1µm to 3µm in size, are optimum for this purpose. However, only about 10 to 20% of the amount of particulate drug dispensed by inhalers is delivered in the range. Large molecule drugs, such as peptides and proteins which are now possible due to genetic engineering, do not pass easily through the airway surface because it is lined with a ciliated mucus-covered cell layer of some depth, making it highly impermeable.

The alveoli in the lungs, however, have a thin single cellular layer as previously described, enabling absorption into the bloodstream. The alveoli are the door to the arterial blood and are at the base of the lungs. So, to reach the alveoli, a particulate drug must be administered in small size particles, and the inhalation must be moderated, i.e., slow and deep. Large particles will impact in the oropharyngeal area or settle in the upper bronchi. If the particles are too small and/or ultralight, they will be exhaled (the last is especially true if air is the tidal front of gas entraining the ultralight particles).

The use of air as a current standard of practice regarding the gas that is inhaled, determines in large part the current state of the art and approaches as to the smallest and lightest particles that can be delivered. The first major opportunity for the loss of administered drug powder or liquid aerosol particles by impaction is in the oropharyngeal area. The second group of locations where particles can be lost to impaction are at each of the bifurcations as one goes deeper into the lungs through successive generations of bronchi and bronchioles.

The large passages through which the air and drug particles travel generate turbulence, which also results in the impaction and loss of drug particles. A desired goal is to increase the laminar flow of the gas stream in the larger air passages, so that particles reach the smaller passages where laminar flow is naturally induced. If there are any constrictions in the bronchi or bronchioles, which results in heavier particles being lost due to sedimentation prior to reaching the desired target location of the alveoli.

A method of drug delivery must take into account this range of tidal volume, as well as the variance in inhalation velocity and the point during inhalation the patient inhales the drugs as this can affect in what part of the mass of gases inhaled the drug particles are traveling, i.e., either in the "tidal front" of gas during a full inspiration and being carried deep into the lungs, or, in the middle, and being deposited in a shallow location where transference of the drug to the arterial blood will not occur.

The prior art mainly teaches the use of air as the primary means of fluidizing dry powder drugs. In addition, the prior art, with regard to Metered Dose Inhalers (MDI) or Dry Powder Inhaler (DPI), also teaches the use of air as the exclusive or primary means of conveying fluidized powder or aerosolized liquid drug into the lungs. In the case of MDI's, it is assumed the propellant evaporates as intended and constitutes a very small fraction of the total gas inhaled at full tidal volume with the drug dose and air. The prior art also discloses the use of a combination of helium and oxygen in nebulizers but the helium content never exceeded 80% of the propellant gas used. This use of air or a combination of helium (less than 80%) and oxygen limits the properties of gas flow when inhalers are used which does not result in a laminar flow thus affecting the ability to deliver a drug to its desired target site for maximum effectiveness.

### SUMMARY OF THE INVENTION

The present invention relates to the use of more than 80% helium or neon in the gas propellant of aerosol inhalers to improve laminar flow. The present invention also relates to a method of treating a subject with a pharmaceutical agent, comprising administering the pharmaceutical agent to the subject's lungs using an aerosol inhaler containing a gas propellant, wherein said gas propellant contains more than 80% helium or neon.

### DETAILED DESCRIPTION OF THE INVENTION

It has now been found that the use of a portable high pressure compressed gas source of more than 80% helium or neon results in improved laminar flow when used as a propellant gas in an aerosol inhaler to fluidize a dry powder pharmaceutical agent is or aerosolize a liquid drug. Preferably, the content of helium or neon in the propellant gas is at least 85%, more preferably at least 90%, and most preferably 100%.

The objective with any method and technology involving inhalers is to a) to generate particles of the optimum size range for deep lung delivery, and b) to get any particles administered past the large airways where they will be lost to turbulence and impaction and into the middle (for treating respiratory diseases) and deep lung (for delivery drugs to the target area where they can enter the arterial blood). Any variability in the dose deposited in the lungs, and where it is deposited in the lungs, could have a major effect on treatment because of the narrow therapeutic range of many drugs, and the potency of such drugs. One well known example is insulin.

The present invention provides for the use of a compact and portable drug inhalation system for delivering dry powder drug formulations in particular, and liquid drug formulations, into the deep lung. Such device uses a pressurized source comprising over 80% helium or neon gas to fluidize dry powder drug formulations and aerosolize liquid drug formulations generating optimal particle sizes and particle sized distributions for delivery of the drug into the lungs for therapy of respiratory and systemic diseases, and in particular deep into the lungs to the alveoli, where the drug can cross the alveolar capillary membrane and enter the arterial blood going directly back to the heart.

Helium or neon with their low molecular weights were chosen as the source of dry powder drug fluidization, liquid drug formulation aerosolization, and drug delivery agent for the invention.

Helium has a density that is only 14% of nitrogen and 12% of oxygen. That means helium can transit rapidly from turbulent flow to laminar flow within the short distance from the inhaler into the oropharyngeal area and to the back of the throat. This is a critical advantage that contributes to the drug delivery aspects of the invention. Turbulent flow is important for fluidizing and rapid mixing of a dry powder drug formulation. Turbulent flow has a uniform velocity profile because of rapid momentum transfer. The powdered medicine can be mixed uniformly when they are shot into the spacer using 100% helium gas, to maintain the 100% helium gas environment created in the spacer prior to injection of the fluidized powder.

However, laminar flow is more preferred once the drug enters the oropharyngeal area and especially the throat, because a high percentage of the drug can be lost due to the turbulence induced impact of particles on the back and walls of the throat entrance (i.e. the "curve" leading from the mouth into the trachea).

A laminar flow retains higher velocity at the center of the flow stream thereby retaining more of the solid particles in the midstream. Thus, laminar flow reduces turbulence and fewer particles will be transported from the center of the gas flow to the outer border of the air flow, reducing the quantity which would impact on the back of the throat and the sidewalls of the throat, trachea, and larger bronchi in the upper lung.

In a CFC based inhaler, the vaporizing CFC driven by its own vapor pressure will result in a substantial drop in temperature of the vaporizing cloud. Temperature drops of 40-50'F are possible. Using compressed gas (over 80% helium or neon), the temperature will drop as it expands and drops in pressure. However, helium has an extremely high heat capacity. This keeps the temperature drop within a few degrees of the room temperature. This avoids the "gagging reflex" that can be induced by cold Freon gas hitting the back of the throat from Metered Dose Inhalers (MDI's). The gagging reflex negates the delivery of a prescribed dose, as the patient stops inhaling before a full and deep inhalation of the aerosolized medication occurs.

Once the powdered medication is fully fluidized and suspended by a high velocity helium-gas stream, the critical task is then reducing the velocity to an acceptable level for inhalation and settling out the heavier particles which one does not want to inhale. This is accomplished using a spacer, which is filled with helium prior to injection of the fluidized dry powder or aerosolized liquid drug formulation.

The spacer reduces the velocity of the gas stream to the acceptable of a "cloud" of particles, the undesirable large particles settle out, and the resulting cloud of remaining particles that fall more into the desired particle size range can be inhaled, where the laminar flow shelves aid in the introduction of a laminar flow out of the spacer of the helium gas and entrained particles.

Then, upon inhalation, it is highly desirable after inhalation to keep the particles of the desired size range from settling. With viscous drag greater than the gravitational settling velocity, the fine solid particles can be suspended indefinitely without settling. On the other hand, additional viscous drag will cause excess pressure drop. It is therefore, desirable to control the viscosity.

The ability of this invention to generate initial high turbulent flow and provide rapid flow deceleration is important to the performance of this inhaler for powdered drug delivery. In contrast to the minimum flow resistance in asthma treatments, the objective of this invention is to deliver the dry powder in a finely dispersed form with minimum powder loss. For example, the friction factor will increase by 5 times when the Reynolds Number is reduced from a turbulent flow of 1 x 10⁵ to a laminar flow of 1.4 x 10³.

Minimal powder loss means less nominal drug needs to be loaded into the device. Because genetically engineered peptides, proteins and hormones are very expensive, this is an important cost reduction factor in the manufacture of the pharmaceutical doses, and the cost to the patient for the therapy.

This can be accomplished despite the presence of a drop in pressure as the gas front moves deeper into the lungs and/or encounters an obstruction. It can also be accomplished in the presence of flow resistance, as in the case of obstructions in asthma or other chronic obstructive pulmonary diseases. The rapid flow transition is made possible by the contribution of the helium and neon molecules. The high thermal conductivity of helium allows temperature rise to propagate rapidly from the mouth wall to the thermal boundary layer next to the surface.

As viscosity increases with temperature in gases (opposite of that of liquid), the viscous drag along the sidewall will slow the gas flow to form the parabolic profile of a laminar flow. Therefore, helium and neon's physical properties are contributing to the turbulent-to-laminar transition. The laminar flow is also important or carrying the fine particulate dry powder or liquid drug formulation to the small airways of the lower lung for systemic delivery. Once the mixture of the helium gas and entrained particles of drug enter the terminal bronchioles and alveoli, the diffusion effect starts to dominate the mass transfer mechanism vs. impaction or sedimentation. This is especially true for smaller and lighter or ultralight particles which may be sub-micron in size.

Helium and neon have molecular weights of only 4 and 10, respectively, verses 28 for nitrogen and 32 for oxygen. For a given volume of gas mixture inside a narrow passage in the lower lung, the helium and neon gases would contain a lot more molecules.

For diffusion dominated deposition, very little gas movement occurs except for molecule exchanges. The very fine particles at this stage (0.5 to 5 microns) can be transported only through molecular diffusion and random molecular motions. Once again, the helium becomes an important factor as the small molecules diffuse forward, dragging the fine medical powder with it through viscous drag.

Larger particles are deposited in the upper lung. The gas velocity will drop to zero. Very fine particles, e.g., smaller than 1 micron, may be fully suspended in the helium without gas movements. At this stage, the viscous drag equalizes the terminal settling velocity of the particles.

Therefore, one can take advantage of this phenomenon to prepare micronized dry powder drug formulations and liquid aerosol nozzles so that particles less then 1 micron in diameter are delivered into the lungs, which if they dominate the mix of particles delivered, reduces the nominal drug quantity that must be loaded into the device to assure an effective dose is delivered to the target site(s) in the deep lung for systemic therapy. Treatment of respiratory diseases can utilize larger particle sizes, as it may not be necessary for them to reach the alveoli.

The small molecular size, high thermal conductivity and high heat capacity are the unique combined proper-ties of helium and neon. They are not the properties of oxygen in the context of helium-oxygen (helium content 80% or less) mixtures, which limited prior art and medical literature have focused on re: delivery of particles or pre-treatment prior to delivery of asthma bronchodilators from an MDI.

In powered drug delivery, the oxygen is added only for safety precautions. The helium required for pushing the powdered drug out of the unit dose tube in the multidose barrel of the invention containing multiple tubes, is between 30cc-70cc, which is injected in one continuous stream. This is added to the 230cc to 270 cc of helium gas injected into the spacer prior to injection of the helium and entrained drug particles. It is understood that the original air and carbon dioxide inside the lung is going to dilute the helium.

The use of a pressurized source of gas provides several advantages over both existing Drug Powder Inhalers (DPI 's) and MDI's. Existing DPI's use the patient's inhalation alone, the patient's inhalation assisted by a propeller, or compressed air generated by a hand pump in a DPI, to fluidize the dry powder drug formulation. One DPI also uses compressed air in a plastic pillow that contains the dry powder drug formulation as an aid to fluidization.

### Drug Powder Inhalers

The present invention offers several advantages over these approaches to fluidizing a dry powder drug formulation. First, the volume of gas and its pressure are independent of the operator's inhalation velocity, ability to generate a given level of inhalation velocity if as in some devices a minimum threshold is required for release of the powder for fluidization, or physical motion. No batteries need to be checked and replaced periodically as is required for the propeller driven system. Compressed air does not have to be pumped prior to each dosing.

Since Inhalers are most often used by children and older patients, a small factory produced compressed gas source eliminates the variability of children following instructions, and the difficulty of arthritic older persons having to try to use a small hand pump mechanism.

A factory produced compressed gas source (over 80% helium or neon) such as a 15 to 90 gram cartridge provides a consistent pressure and volume of gas which in turn generates a constant and standardized fluidization of a dry powder drug formulation for a large number of doses from each cartridge.

DPI's that rely on inhalation power, a propeller or hand pumped compressed air, all use air that is from the environment the user is standing in. If the air is humid, it can cause clumping of the micronized dry powder drug formulation, resulting in larger particles that may not reach the upper lung, let alone the deep lung.

A factory produced compressed gas source (over 80% helium or neon) can be produced as a desiccated dry gas, eliminating this problem in humid climates, which make up a large part of the potential geographic patient market. A factory produced source of pressurized gas (over 80% helium or neon) also provides the advantage a high velocity gas stream, which provides the advantage of a more forceful impact on and fluidization of a dry powder drug formulation, compared to the force generated by inhaled air, battery powered propeller assisted air, or hand pumped compressed air. The result is that the powder can be fluidized and deagglomerated more completely, with the result being more consistent and effective use of a unit dose of the formulation, and potentially a reduction in the nominal drug powder formulation that must be loaded as more is consistently deliverable.

Where prior art uses blisters and reservoirs of powder, the present invention uses tubes containing powder. The use of a tube allows the effect of the gas pressure to be magnified in terms of velocity generated and impact of the gas on the particles. Furthermore, the impact of the high pressure helium or neon "front" of gas hitting the powder is more effective at fluidizing the powder then inhalers that operate using the patient's variable inhalation force (pulling vs. blowing the powder apart), or, the weaker force of propeller driven or hand pumped compressed air.

Because compressed gas like helium or neon is prepared at a factory and put into cartridges, it can be desiccated and remain so in its cartridge prior to use. This is an advantage over all other inhalers that use ambient air, whether inhaled, driven by a propeller or compressed with a small hand pump that is part of the inhaler, because ambient air, depending on geographic location and/or season, can be laden with varying degrees of humidity. This in turn, would cause variability in the fluidization, deagglomeration and post clumping of dry powder drug formulations, which in turn effects the fine particle fraction available for pulmonary administration and effective therapy.

### Metered Dose Inhalers

Another advantage of the compressed low molecular weight 100% helium gas is that it can also be used for delivering liquid drug formulations. Compressed helium is a much better liquid aerosolizing/atomizing agent because of its high velocity of release and it does not have the same cooling characteristics of liquid CFC's.

In the case of the invention, the multidose insert containing multiple sealed unitdoses of liquid drug, or a reservoir multidose liquid drug source, is stored separately from the compressed gas.

In MDI's, the propellant and drug formulation are stored together, along with many other additional additive ingredients. In the case of liquid drug suspension formulations, the MDI must be shaken before each use to try and achieve a uniform consistent dosing. In the case of a drug in solution, temperature changes can make the drug compound, which is packaged with the propellant, come out of solution.

Aerosol administration is useful for a variety of drugs. Whereas historically drugs administered to the lungs were to treat respiratory disease, the focus is now on administering drugs via the pulmonary route to treat other systemic diseases and conditions. The current focus is also on drugs to treat systemic rather then just respiratory diseases and conditions. This requires delivery of the drug to that part of the lung, the alveoli, where it can be absorbed into the arterial blood supply.

Of special interest is the ability to deliver large molecules, such as proteins or peptides in the form of fluidized or atomized particulate aerosols with particles 1-3 microns in diameter, while in their most stable state of a dry powder. Examples of classes of drugs which may be formulated for pulmonary administration, include those for chronic obstructive lung diseases such as the classes of agents commonly referred to as anicholinergic agents, beta-adrenergic agents, corticosteroids, antiproteinases, and mucolytics.

Other therapeutic pharmaceuticals for respiratory disease used in dry powder and/or form which can be used by the present invention include but are not limited to benzamil, phenamil, isoproterenol, metaproterenol, beta 2 agonists in general, proctaterol, salbutamol, fenoterol, ipratropium, fulutropium, oxitropium, beclomethasone dipropionate, fluticasonepropionate, salmeterol xinafoate, albuterol, terbutaline sulphate, budesonide, beclomethasone dipropionate monohydrate, surfactants such as colfosceril palmitate, cetylalcohol and tyloxapol, P2Y₂ agonist (rapid stimulates mucus and is potentially for use in COPD and CF), aerosolized dextran (for CF), and mannitol powders (for bronchial provocative challenge). An example of another therapeutic drug that could be delivered by the invention is pentamidine for AIDS related therapy.

Examples of proteins and peptide hormone drugs that may be administered with the invention, which may or not be glycosolated, include somatostatin, oxytocin, desmopressin, LHRH, nafarelin, leuprolide, ACTH analog, secretin, glucagon, calcitonin, GHRH, growth hormone, insulin, parathyhroid, estradiol and follicle stimulating hormone and prostaglandin El.

In addition, genes, oligonucleotides, anticoagulants such as heparin and tPA, anti-infectives to treat localized and systemic bacterial or fungal infections, enzymes, enzyme inhibitors, vaccines, anesthetics, pain killers, and agents that can turn certain types of receptors on, off, or enhance their response, are possible therapeutic drugs or action inducing substances which may be delivered using the present invention.

Ergotamine for the treatment of migraine headaches and nicotine to substitute for and eventually eliminate cravings for tobacco, is also therapeutic formulation which maybe administered by the invention. Furthermore, controlled release drugs such as those that are liposome based and which are designed for pulmonary drug delivery to treat respiratory and systemic diseases over a period of time due to the chronic nature of the illness or the mode in which the illness responds to medication, or the mode in which the medication operates, may be administered by the invention.

The invention has been described in terms of preferred embodiments thereof, but is more broadly applicable as will be understood by those skilled in the art. The scope of the invention is limited only by the following claims.

## Claims

1. In an aerosol inhaler, the improvement comprising the use of a gas propellant, wherein said gas propellant contains more than 80% helium or neon.

2. The aerosol inhaler of claim 2, wherein the gas propellant contains at least 85% helium.

3. The aerosol inhaler of claim 2, wherein the gas propellant contains at least 90% helium.

4. The aerosol inhaler of claim 2, wherein the gas propellant is helium.

5. The aerosol inhaler, wherein the gas propellant contains more than 80% neon.

6. The aerosol inhaler of claim 1, wherein the gas propellant further contains oxygen.

7. A method of treating a subject with a pharmaceutical agent, comprising administering the pharmaceutical agent to the subject's lungs using an aerosol inhaler containing a gas propellant, wherein said a gas propellant contains more than 80% helium or neon.

8. The method of claim 7, wherein the gas propellant contains more than 80% helium.

9. The method of claim 7, wherein the gas propellant contains more than 80% neon.

10. The method of claim 7, wherein the gas propellant further contains oxygen.
